# EUROPEAN PATENT APPLICATION

(11) **EP 1 148 099 A2**
(43) Date of publication of application: **24.10.2001**
(21) Application number: 01303293.3
(22) Date of filing: 06.04.2001
(51) Int. Cl.: C08L 83/04, C08G 77/04, C08G 77/12, A61K 7/46

(54) **Organopolysiloxane composition and method of preparation**

(30) Priority: 17.04.2000 JP 2000114705
(71) Applicant: Dow Corning Toray Silicone Co., Ltd., Tokyo (JP)
(72) Inventor: Kondo, Hidetoshi, Dow Corning Toray Silic. Co.Ltd., Ichihara-shi, Chiba Prefecture (JP); Takahashi, Masahiro, Dow Corn.Toray Silic. Co.Ltd., Ichihara-shi, Chiba Prefecture (JP)
(74) Representative: Kyle, Diana

(57) **Abstract**

An organopolysiloxane composition comprising 5 to 99.9 wt% of a crosslinked product of a block copolymer consisting of an organopolysiloxane block and a polyoxyalkylene block bonded to silicon atoms and described by the following general formula -R¹(OR²)ₘOR¹-, where R¹ and R² are independently selected alkylene groups, and *m* is a positive integer and 95 to 0.1 wt% of a fragrance material; the aforementioned organopolysiloxane composition emulsified in water; and a method of preparing the above compositions.

## Description

The present invention relates to an organopolysiloxane-type composition containing a fragrance material and to a method for preparing the same. More specifically the present material relates to an organopolysiloxane composition possessing a superior fragrance retention capability and compounding stability, and to a method for preparing the same.

Organopolysiloxane compositions containing fragrance materials are known. Examples of such compositions include an organopolysiloxane composition comprising a cured product of a room temperature curable organopolysiloxane composition and a fragrance material (see JP-A Sho 61-13961), an organopolysiloxane composition comprising silicone rubber particles, silicone oil, and a fragrance material (see JP-A Hei 10-036228), and an organopolysiloxane composition comprising silicone oil and a fragrance material (see JP-A Hei 11-114042). The problem with these known fragrance containing compositions is their insufficient fragrance retention when added, for example, to cosmetic materials, detergents, lustering agents, surface finishing agents, fiber treating agents, and coating materials.

It is an object of the present invention to provide an organopolysiloxane-type composition which is superior in fragrance retention and exhibits excellent compounding stability when used as an additive for cosmetic materials, detergents, lustering agents, surface finishing agents, fiber treating agents, coating materials, etc., and, furthermore, which can impart excellent skin feel, finish properties, surface smoothness characteristics, and surface-protecting properties to the resultant compounds, as well as to provide a method for preparing the same.

The present invention relates to an organopolysiloxane composition comprising (A) 5 to 99.9 wt% of a crosslinked product of a block copolymer consisting of an organopolysiloxane block and a polyoxyalkylene block bonded to silicon atoms and described by the following general formula -R¹(OR²)ₘOR¹-, where R¹ and R² are independently selected alkylene groups, and *m* is a positive integer and (B) 95 to 0.1 wt% of a fragrance material; the aforementioned organopolysiloxane composition emulsified in water; and a method of preparing the above compositions.

The present invention relates to an organopolysiloxane composition comprising (A) 5 to 99.9 wt% of a crosslinked product of a block copolymer consisting of an organopolysiloxane block and a polyoxyalkylene block bonded to silicon atoms and described by the following general formula -R¹(OR²)ₘOR¹-, where R¹ and R² are independently selected alkylene groups, and *m* is a positive integer and (B) 95 to 0.1 wt% of a fragrance material; the aforementioned organopolysiloxane composition emulsified in water; and a method of preparing the above compositions.

First of all, a detailed explanation will be provided regarding the organopolysiloxane composition of the present invention.

The crosslinked product of a block copolymer is composed of an organopolysiloxane block and a polyoxyalkylene block described by general formula ―R¹(OR²)ₘOR¹ which is bonded to silicon atoms of the organopolysiloxane. In the above formula, R¹ and R² are independently selected alkylene groups exemplified by ethylene, propylene, butylene, isobutylene, pentamethylene, octamethylene, decamethylene, dodecamethylene, and cyclohexylene. Among these, ethylene, propylene, and butylene are preferable. The subscript *m* is a positive integer, preferably in the range of from 1 to 100, and even more preferably in the range of from 20 to 80. It is preferable that the polyoxyalkylene blocks be described by the following general formula―R¹(OC₂H₄)ₚ(OC₃H₆)_{q}OR¹, where R¹ is the same as above, subscript *p* is 0 or a positive integer preferably in the range of from 0 to 20, and subscript *q* is a positive integer preferably, in the range of 20 to 80, with the proviso that *p* is smaller than *q* at all times. In addition, when *p* is a positive integer, the bond between oxyethylene and oxypropylene may be a block bond or a random bond. Specifically, groups represented by the following formulas are suggested as examples.

―(CH₂)₃(OC₂H₄)₁₀(OC₃H₆)₅₀O(CH₂)₃―

―(CH₂)₃(OC₃H₆)₅₀O(CH₂)₂―

―(CH₂)₃(OC₃H₆)₃₀O(CH₂)₃―

―(CH₂)₃(OC₂H₄)₅(OC₃H₆)₃₀ O(CH₂)₂―

―(CH₂)₂(OC₂H₄)₅(OC₃H₆)₅₀ O(CH₂)₂―

―(CH₂)₂(OC₃H₆)₂₀O(CH₂)₂―.

In addition, in the crosslinked product, silicon-bonded groups other than the above-mentioned polyoxyalkylene blocks are exemplified by substituted or unsubstituted monovalent hydrocarbon groups, such as methyl, ethyl, propyl, butyl, pentyl, hexyl, heptyl, octyl, nonyl, decyl, hexadecyl, heptadecyl, octadecyl, and other alkyl groups; cyclopentyl, cyclohexyl, and other cycloalkyl groups; phenyl, tolyl, xylyl, and other aryl groups; benzyl, phenethyl, and other aralkyl groups; 3-chloropropyl, 3,3,3-trifluoropropyl, and other halogenated alkyl groups. In addition to these groups, a small amount of methoxy, ethoxy, propoxy, and other alkoxy groups and hydroxyl groups may be present. The aforementioned crosslinked product at room temperature may be in a liquid, gel-like, or elastomer state. The liquid or gel-like state in which the product possesses flowability is preferable. Also, the term "liquid," as used in the present invention, indicates a substance with a viscosity in the range of from 1 mPa·s to 10,000,000 mPa·s at normal temperature, a viscosity in the range of from 100,000 mPa·s to 10,000,000 mPa·s being particularly preferable. In addition, the term "gel-like" indicates a property where a non-flowable substance undergoes an irreversible deformation to a flowable substance when an external force is applied thereto.

Preferred is when the aforementioned block copolymer composition is a crosslinked product of a hydrosilation reaction comprising the reaction product of (a) an organopolysiloxane having at least two silicon-bonded hydrogen atoms in one molecule; (b) a polyoxyalkylene described by general formula R⁴(OR²)ₘOR⁴, where R² and *m* are the same as above and each R⁴ is an independently selected alkenyl group, optionally (c) an organopolysiloxane having at least two silicon-bonded alkenyl groups in one molecule, and (d) a catalytic amount of a hydrosilation catalyst.

Specifically suggested is a crosslinked product obtained via a hydrosilation reaction between the above-mentioned components (a) and (b) or between the above-mentioned components (a), (b), and (c), with the crosslinked product of a crosslinkable organopolysiloxane composition comprising the above-described components (a) to (d) particularly preferred. In addition, although crosslinking is conducted at room temperature, if necessary it may be carried out under heating.

Component (a) is crosslinked via a hydrosilation reaction with component (b) and optionally component (c). In component (a) silicon-bonded groups other than hydrogen atoms are exemplified by substituted or unsubstituted monovalent hydrocarbon groups, such as methyl, ethyl, propyl, butyl, pentyl, hexyl, heptyl, octyl, nonyl, decyl, hexadecyl, heptadecyl, octadecyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these groups, a small amount of methoxy, ethoxy, propoxy, and other alkoxy groups and hydroxyl groups may be present. There are no limitations concerning the structure of component (a) and suggested structures include, for example, linear, partially branched linear, branched, or cyclic structures; with linear or partially branched linear structures being preferable. There are no limitations concerning the viscosity of component (a) at 25°C, however preferably it should be in the range of from 1 mPa·s to 100,000 mPa·s and even more preferably in the range of from 1 mPa·s to 10,000 mPa·s. Component (a) is exemplified by dimethylpolysiloxane having both terminal ends of the molecular chain blocked by dimethylhydrogensiloxy groups, a copolymer of methylhydrogensiloxane and dimethylsiloxane having both terminal ends of the molecular chain blocked by dimethylhydrogensiloxy groups, and a copolymer of methylhydrogensiloxane and dimethylsiloxane having both terminal ends of the molecular chain blocked by trimethylsiloxy groups; and by organopolysiloxanes obtained by substituting, for example, phenyl, ethyl, lauryl, stearyl, and 3,3,3-trifluoropropyl for some of the methyl groups in the above-mentioned polysiloxanes.

Component (b) of the present invention improves the retention of the fragrance material (i.e. durability of the fragrance) in the present composition. In the above-described component (b) described by formula R⁴(OR²)ₘOR⁴, where R², R⁴, and *m* are the same as above. Each R⁴ is an independently selected alkenyl group exemplified by vinyl, allyl, butenyl, pentenyl, hexeny, octenyl, and decenyl. Preferred is when R⁴ is vinyl and allyl. It is preferable that component (b) be described by general formula R⁴(OC₂H₄)ₚ(OC₃H₆)_{q}OR⁴, where *p* and *q* are the same as above. Specifically, oxyalkylene compounds described by the following formulas are suggested as examples:

CH₂=CHCH₂―(OC₂H₄)₁₀(OC₃H₆)₅₀O―CH₂CH=CH₂

CH₂=CHCH₂―(OC₃H₆)₅₀O―CH=CH₂

CH₂=CHCH₂―(OC₃H₆)₃₀O―CH₂CH=CH₂

CH₂=CHCH₂―(OC₂H₄)₅(OC₃H₆)₃₀ O―CH=CH₂

CH₂=CH―(OC₂H₄)₅(OC₃H₆)₅₀ O―CH=CH₂

CH₂=CH―(OC₃H₆)₂₀ O―CH=CH₂

The amount of added component (b) is in the range of 0.01 to 100 parts by weight, preferably in the range of 0.1 to 100 parts by weight, and especially preferably in the range of 1 to 80 parts by weight per 1 part by weight of component (a). This is due to the fact that when the amount of added component (b) is less than 0.01 parts by weight, the fragrance retention capability of the present composition tends to decrease, and when it exceeds 100 parts by weight the skin feel, finish properties, surface smoothness characteristics, surface-protecting properties, and other characteristics of the resultant compounds tend to deteriorate when the present composition is added to, for example, cosmetic materials, detergents, lustering agents, surface finishing agents, fiber treating agents, and coating materials.

The organopolysiloxane of component (c) is an optional component which has at least two silicon-bonded alkenyl groups in one molecule. The alkenyl groups are exemplified by vinyl, allyl, butenyl, pentenyl, and hexenyl. In this organopolysiloxane, silicon-bonded groups other than alkenyl groups are exemplified by substituted or unsubstituted monovalent hydrocarbon groups, such as methyl, ethyl, propyl, butyl, pentyl, hexyl, heptyl, octyl, nonyl, decyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these groups, a small amount of methoxy, ethoxy, propoxy, and other alkoxy groups and hydroxyl groups may be present. Suggested structures for component (c) include linear, partially branched linear, branched, or cyclic structures; with linear or partially branched linear structures being especially preferred. There are no limitations concerning the viscosity of component (c) at 25°C, preferably however it should be in the range of from 10 mPa·s to 100,000 mPa·s and even more preferably in the range of from 10 mPa·s to 10,000 mPa·s. Component (c) is exemplified by dimethylpolysiloxane having both terminal ends of the molecular chain blocked by dimethylvinylsiloxy groups, a copolymer of methylvinylsiloxane and dimethylsiloxane having both terminal ends of the molecular chain blocked by dimethylvinylsiloxy groups, and a copolymer of methylvinylsiloxane and dimethylsiloxane having both terminal ends of the molecular chain blocked by trimethylsiloxy groups, and by organopolysiloxanes obtained by substituting, for example, phenyl, ethyl, lauryl, stearyl, and 3,3,3-trifluoropropyl for some of the methyl groups in the above-mentioned siloxanes. The amount of added component (c) is in the range of 0 to 100 parts by weight, preferably in the range of 0 to 80 parts by weight, and especially preferably in the range of 0 to 50 parts by weight per I part by weight of component (a). This is due to the fact that when the amount of added component (c) exceeds 100 parts by weight, the fragrance retention capability of the present composition tends to decrease.

The hydrosilation reaction catalyst of component (d) is used to promote a hydrosilation reaction between the above-described components (a) and (b) or (a) to (c). Platinum catalysts, rhodium catalysts, and palladium catalysts are suggested as such catalysts. Among these, platinum catalysts are preferred such as chloroplatinic acid, alcohol solutions of chloroplatinic acid, olefin complexes of platinum, alkenylsiloxane complexes of platinum, carbonyl complexes of platinum, platinum black, platinum catalysts supported on silica, as well as their mixtures. The amount of added component (d) is a catalytic amount and when a platinum catalyst is used the amount is preferably such that the concentration of platinum metal is within the range of 0.01 to 1,000 parts by weight per 1,000,000 parts by weight of the total of components (a) and (b) or (a) to (c). This is due to the fact that when the platinum concentration is less than 0.01 parts by weight, the hydrosilation reaction does not proceed sufficiently to completion and when an amount exceeding 1,000 parts by weight is added the effect of promoting the hydrosilation reaction is not increased.

The fragrance material of component (B) can be natural fragrance materials, synthetic fragrance materials, or mixed fragrance materials. With account taken of their solubility in the crosslinked product of the aforementioned block copolymer, component (B) is specifically exemplified by hexanol, heptanol, octanol, nonanol, decanol, *cis*-3-hexenol, and other aliphatic alcohols; hexanal, heptanal, octanal, nonanal, decanal, 10-undecenal, and other aliphatic aldehydes; 2-octanone, methylheptenone, and other aliphatic ketones; isopentyl acetate, *cis*-3-hexenyl acetate, allyl cyclohexylpropionate, and other aliphatic esters; isoparaffin, and other aliphatic hydrocarbons; D-limonene, P-cymene and other terpene-series hydrocarbons; linalool, terpineol, citronellol, and other terpene-series alcohols; citral, citronellal, and other terpene-series aldehydes; camphor, L-carbone, menthone, and other terpene-series ketones; geranyl acetate, linalyl propionate, citronellyl isobutyrate, and other terpene-series esters; rose oxides, linalool oxides, and other terpene-series ethers; lemon oil, orange oil, lime oil, and other citrus essential oils; lavender oil, rosemary oil, peppermint oil, and other herbal essential oils; rose oil, neroli oil, and other floral essential oils; and cyclopentadecanolide and other synthetic musk fragrances. Such fragrance materials can be used singly or as a mixture of two or more materials. In addition, these fragrance materials are preferably liquid at normal temperature.

The proportion, in which the above-described (A) crosslinked product of the block copolymer and the (B) fragrance material are compounded is in the range of 5 to 99.9 : 95 to 0.1 wt%, preferably in the range of from 25 to 99.9:75 to 0.1 wt%, and even more preferably in the range of 40 to 99.9:60 to 0.1 wt%. This is due to the fact that when the content of the fragrance material is less than 0.1 wt%, the fragrance cannot be imparted when the present composition is added to cosmetic materials, detergents, lustering agents, surface finishing agents, fiber treating agents, coating materials, and the like, and when it exceeds 95 wt% the fragrance retention capability of the present composition tends to decrease.

Although there are no limitations concerning the form of the present composition, an emulsion produced by emulsification in water is preferred. It is desirable to use a surface active agent in the preparation of the emulsion in order to improve the emulsion stability of the crosslinkable organopolysiloxane composition. The surface active agents to be used are exemplified by anionic surface active agents, such as hexylbenzenesulfonic acid, octylbenzenesulfonic acid, decylbenzylsulfonic acid, dodecylbenzenesulfonic acid, cetylbenzenesulfonic acid, myristylbenzenesulfonic acid and their salts; cationic surface active agents, such as octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow trimethylammonium hydroxide, coconut oil trimethylammonium hydroxide; nonionic surface active agents of the polyester series, as well as ethylene oxide adduct of diethylene glycol trimethyl nonanol, polypropylene glycol, polyethylene glycol, polyoxyalkylene sorbitan ester, polyoxyalkylene alkyl ester, polyoxyalkylene alkyl phenol, and polyoxyalkylene alkyl ether. Such surface active agents can be used singly or as a mixture of two or more agents. The use of nonionic surface active agents for emulsification is particularly preferable when using the present compositions as additives for cosmetic products. There are no limitations concerning the amount of compounded surface active agent, but preferably it is in the range of 0.01 to 50 parts by weight, and even more preferably in the range of 0.1 to 20 parts by weight per 100 parts by weight of the present composition. This is due to the fact that when the surface active agent is less than 0.01 parts by weight the stability of the emulsion decreases, and on the other hand when it exceeds 50 parts by weight it adversely affects the characteristics of the resultant compounds when the composition is added to cosmetic materials, detergents, lustering agents, surface finishing agents, fiber treating agents, coating materials, and the like. In addition, although there are no limitations concerning the amount of added water preferably it is in the range of 10 to 1,000 parts by weight per 100 parts by weight of the present composition. This is due to the fact that when the water is less than 10 parts by weight the stability of the emulsion decreases, and on the other hand when it exceeds 1,000 parts by weight sufficient fragrant properties cannot be imparted to cosmetic materials, detergents, lustering agents, surface finishing agents, fiber treating agents, coating materials, and the like.

Next, a detailed description will be provided regarding the method of preparing the present organopolysiloxane composition. A method in accordance with which (A) 5 to 99.9 wt% of a crosslinkable organopolysiloxane composition comprising the above-described components (a) to (d) and (B) 95 to 0.1 wt% of a fragrance material are subjected to crosslinking in an emulsified state in water, or a method in accordance with which the fragrance material is added after crosslinking a crosslinkable organopolysiloxane composition comprising the above-described components (a) to (d) in an emulsified state in water are suggested as the preparative methods of the present composition.

In the former method, a process, in which the above-described components (a) and (b) and the fragrance material, or (a), (b), and (c) and the fragrance material are emulsified into an emulsion in advance and then component (d) is added thereto, is preferable as the process to be used for preparing the emulsion of the crosslinkable organopolysiloxane composition and fragrance material. It is preferable to use colloid mills, homogenizers, homomixers, and other emulsifying equipment, as well as high-shear agitators used for high-viscosity liquids. At such time, it is preferable to admix the above-described surface active agents, the use of nonionic surface active agent being especially preferable. Although the crosslinking reaction after emulsification proceeds even at room temperature, if necessary the emulsion may be heated.

In addition, in the latter method, a process, in which an emulsion is prepared by emulsifying the above-described components (a) and (b) or (a), (b), and (c) components in water in advance and then component (d) is added to the emulsion, is preferable as the process to be used for preparing the emulsion of the crosslinkable organopolysiloxane composition. It is preferable to use colloid mills, homogenizers, homomixers, and other emulsifying equipment, as well as high-shear agitators used for high-viscosity liquids. At such time, it is preferable to admix the above-described surface active agents, the use of nonionic surface active agent being especially preferable. Although the crosslinking reaction after emulsification proceeds even at room temperature, if necessary, the emulsion may be heated. The fragrance material is added after preparing an aqueous emulsion of the crosslinked product of the block copolymer in this manner, with the mixing ratio of the crosslinked product of the block copolymer and the fragrance material being in the range of 5 to 99.9 : 95 to 0.1 wt%.

Because the present organopolysiloxane composition has superior fragrance retention capability when it is used as an additive for various cosmetic materials, detergents, lustering agents, surface finishing agents, fiber treating agents, coating materials, and the like, the products are characterized by retaining the fragrance over an extended period of time. Furthermore, the present composition is superior in compounding stability with respect to various materials and can impart excellent skin feel, finishing properties, surface smoothness characteristics, surface-protective properties, and other characteristics to the resultant products. In addition, although the preparative method of the present invention yields an aqueous emulsion of the present composition, if necessary, water can be removed.

### Application Examples

Hereinbelow, the present invention is explained in detail by referring to application examples. In the application examples, the term "viscosity" refers to a value obtained at 25°C. A liquid mixed fragrance material obtained by blending limonene, cyclohexylaldehyde, and allyl heptate in a proportion of 1:1:1 was used as the fragrance material. In addition, the fragrance retention properties, compounding stability, flexibility, and smoothness were measured in accordance with the measurement methods described below.
- O: Fragrance retention properties
A bundle of hair with a length of 15 cm and a weight of 15g was washed in an aqueous solution of sodium polyoxyethylene alkyl sulfate, rinsed, and then a 1g test sample was applied thereto. The hair was allowed to stand indoors at a temperature of 20°C and a humidity of 40%, and the presence of the scent 1 day, 2 days, and 3 days later was evaluated in the following manner.
A: A distinct scent could be discerned.
B: A faint scent could be discerned.
C: No scent at all could be discerned.
- O: Compounding stability
After allowing the sample to stand for 1 day at 50°C, the state of its external appearance was visually evaluated in the following manner.
A: Uniform.
B: Slight separation detected.
C: Completely separated.
- O: Flexibility & Smoothness
A bundle of hair with a length of 15 cm and a weight of 15g was washed in an aqueous solution of sodium polyoxyethylene alkyl sulfate, rinsed, and then 1 g of a test sample was applied thereto. The hair was allowed to dry indoors and the flexibility and smoothness of the hair was evaluated by tactile sensation in accordance with the following evaluation criteria.
A: Excellent.
B: Rather good.
C: Not very good.
D: No good.

Application Example 1. A mixture comprising 2.4 parts by weight of a copolymer (content of silicon-bonded hydrogen atoms: 0.8 wt%) of dimethylsiloxane and methylhydrogensiloxane having both terminal ends of the molecular chain blocked by trimethylsiloxy groups, which had a viscosity of 15 mPa·s; 39.6 parts by weight of polyoxypropylene described by formula CH₂=CHCH₂(OC₃H₆)₅₀OCH₂CH=CH₂, which had both terminal ends of the molecular chain blocked by an allyl group; and 20 parts by weight of the fragrance material were mixed. The mixture was then combined with 3 parts by weight of polyoxyethylene lauryl ether (HLB=13.0) and 35 parts by weight of water and the contents were emulsified to prepare an emulsion. Subsequently, a solution of a 1,3-divinyltetramethyldisiloxane complex of platinum (platinum metal concentration: 0.04 wt%) was added to, and uniformly mixed with, the emulsion in an amount sufficient to produce a platinum metal concentration of 20 ppm based on the total weight of polyoxypropylene and the copolymer of dimethylsiloxane and methylhydrogensiloxane in the above-mentioned emulsion. Subsequently, the mixture was left to stand at room temperature for 1 day to carry out a hydrosilation reaction and prepare an emulsion of an organopolysiloxane composition. A sample was prepared by combining the obtained emulsion with a hair conditioner prepared from 15 parts by weigh of triethanolamine alkyl ether sulfate, 5 parts by weight of fatty acid monoethanolamide, 2 parts by weight of ethylene glycol monostearate, and 78 parts by weight of water. The emulsion was added in such an amount that concentration of the fragrance material was about 1 Wt.%. The fragrance retention properties, compounding stability, as well flexibility and smoothness of the sample were measured and the results were listed in Table 1. Also, a part of the emulsion had the water removed providing a liquid with a viscosity of 300,000 mPa·s.

Application Example 2. A mixture was formed comprising 3.8 parts by weight of a copolymer (content of silicon-bonded hydrogen atoms: 0.2 wt%) of dimethylsiloxane, octadecylmethylsiloxane, and methylhydrogensiloxane having both terminal ends of the molecular chain blocked by trimethylsiloxy groups, which had a viscosity of 500 mPa·s, the copolymer being described by the following formula 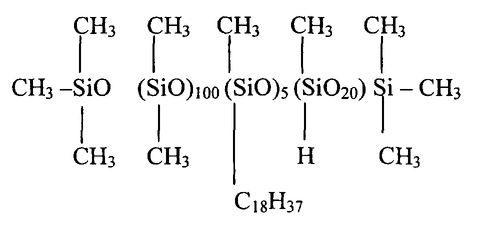 with 24.2 parts by weight of a copolymer of oxypropylene and oxyethylene having both terminal ends of the molecular chain blocked by allyl groups and described by the following formula CH₂=CHCH₂(OC₂H₄)₅(OC₃H₆)₅₀OCH₂CH=CH₂. The mixture was combined with 2 parts by weight of polyoxyethylene- polyoxypropylene-tetradecyl ether (HLB=11.0) and 20 parts by weight of water and the mixture was emulsified. Subsequently, a solution of a 1,3-divinyltetramethyldisiloxane complex of platinum (platinum metal concentration: 0.04 wt%) was added to, and uniformly mixed with, the emulsion an the amount sufficient to produce a platinum metal concentration of 20 ppm based on the total weight of the copolymer of oxyethylene and oxypropylene and the copolymer of dimethylsiloxane, octadecylmethylsiloxane, and methylhydrogensiloxane in the above-mentioned emulsion. Subsequently, the mixture was left to stand at room temperature for 1 day to carry out a hydrosilation reaction and prepare an emulsion of the crosslinked product of the block copolymer. Subsequently, a white uniform emulsion of an organopolysiloxane composition was prepared by slowly adding 50 parts by weight of a fragrance material in a dropwise manner to the mixture under agitation. A sample was prepared by combining the obtained emulsion with a hair conditioner prepared from 15 parts by weigh of triethanolamine alkyl ether sulfate, 5 parts by weight of fatty acid monoethanolamide, 2 parts by weight of ethylene glycol monostearate, and 78 parts by weight of water. The emulsion was added in such an amount that concentration of the fragrance material was about 1 Wt.%. The fragrance retention properties, compounding stability, as well flexibility and smoothness of the sample were measured and the results are listed in Table 1. Also, a part of the emulsion had the water removed providing a liquid with a viscosity of 200,000 mPa·s.

Practical Example 3. A mixture was formed comprising 2.2 parts by weight of a copolymer (content of silicon-bonded hydrogen atoms: 0.8 wt%) of dimethylsiloxane and methylhydrogensiloxane having both terminal ends of the molecular chain blocked by trimethylsiloxy groups, which had a viscosity of 15 mPa·s; 22.3 parts by weight of polyoxypropylene described by formula CH₂=CHCH₂(OC₃H₆)₅₀OCH₂CH=CH₂, which had both terminal ends of the molecular chain blocked by an allyl group; 17.5 parts by weight of dimethylpolysiloxane (content of vinyl groups: 0.5 wt%) having both terminal ends of the molecular chain blocked by dimethylvinylsiloxy groups, which had a viscosity of 400 mPa·s; and 5 parts by weight of the fragrance material. An emulsion was prepared by adding 3 parts by weight of polyoxyethylene lauryl ether (HLB=13.0) and 50 parts by weight of water and emulsifying the mixture. Subsequently, a solution of a 1,3-divinyltetramethyldisiloxane complex of platinum (platinum metal concentration: 0.04 wt%) was added to, and uniformly mixed with, the emulsion in an amount sufficient to produce a platinum metal concentration of 20 ppm based on the total weight of the dimethylpolysiloxane, polyoxypropylene, and copolymer of dimethylsiloxane and methylhydrogensiloxane in the above-mentioned emulsion. Subsequently, the mixture was left to stand at room temperature for 1 day to carry out a hydrosilation reaction and prepare an emulsion of an organopolysiloxane composition. A sample was prepared by combining the obtained emulsion with a hair conditioner prepared from 15 parts by weigh of triethanolamine alkyl ether sulfate, 5 parts by weight of fatty acid monoethanolamide, 2 parts by weight of ethylene glycol monostearate, and 78 parts by weight of water. The emulsion was added in such an amount that concentration of the fragrance material was about 1 Wt.%. The fragrance retention properties, compounding stability, as well flexibility and smoothness of the sample were measured and the results are listed in Table 1. Also, a part of the emulsion had the water removed providing a liquid with a viscosity of 500,000 mPa·s.

Comparative Example 1. An emulsion of an organopolysiloxane composition was prepared by mixing 42 parts by weight of dimethylpolysiloxane having both terminal ends of the molecular chain blocked by trimethylsiloxy groups, which had a viscosity of 1,000 mPa·s, with 5 parts by weight of the fragrance material, followed by adding 3 parts by weight of polyoxyethylene lauryl ether (HLB=13.1) and 50 parts by weight of water and emulsifying the mixture. A sample was prepared by combining the obtained emulsion with a hair conditioner prepared from 15 parts by weight of triethanolamine alkyl ether sulfate, 5 parts by weight of fatty acid monoethanolamide, 2 parts by weight of ethylene glycol monostearate, and 78 parts by weight of water. The emulsion was added in such an amount that concentration of the fragrance material was about 1 Wt.%. The fragrance retention properties, compounding stability, as well flexibility and smoothness of the sample were measured and the results are listed in Table 1. Also, a part of the emulsion was sampled and the water removed providing a liquid with a viscosity of 900 mPa·s.

Comparative Example 2. 5 Parts by weight of a copolymer (content of silicon-bonded hydrogen atoms: 0.06 wt%) of dimethylsiloxane and methylhydrogensiloxane having both terminal ends of the molecular chain blocked by trimethylsiloxy groups, which had a viscosity of 500 mPa·s; 40 parts by weight of a copolymer of dimethylsiloxane and methylvinylsiloxane (content of vinyl groups: 0.5 wt%) having both terminal ends of the molecular chain blocked by dimethylvinylsiloxy groups, which had a viscosity of 400 mPa·s; 5 parts by weight of the fragrance material, and a solution of a 1,3-divinyltetramethyldisiloxane complex of platinum, in an amount sufficient to produce a platinum metal concentration of 20 ppm based on the total weight of the above-mentioned copolymer of dimethylsiloxane and methylhydrogensiloxane and copolymer of dimethylsiloxane and methylvinylsiloxane, were combined and uniformly mixed. Subsequently, an emulsion was prepared by adding 3 parts by weight of polyoxyethylene cetyl ether (HLB=17.0) and 47 parts by weight of water and emulsifying the mixture. An emulsion of an organopolysiloxane composition was then obtained by allowing the emulsion to stand at room temperature for 1 day to carry out a hydrosilation reaction. A sample was prepared by combining the with a hair conditioner prepared from 15 parts by weigh of triethanolamine alkyl ether sulfate, 5 parts by weight of fatty acid monoethanolamide, 2 parts by weight of ethylene glycol monostearate, and 78 parts by weight of water. The emulsion was added in such an amount that concentration of the fragrance material was about 1 Wt.%. The fragrance retention properties, compounding stability, as well flexibility and smoothness of the sample were measured and the results are listed in Table 1. Also, a part of the emulsion was sampled and the water removed thereby providing a liquid with a viscosity of 200,000 mPa·s.

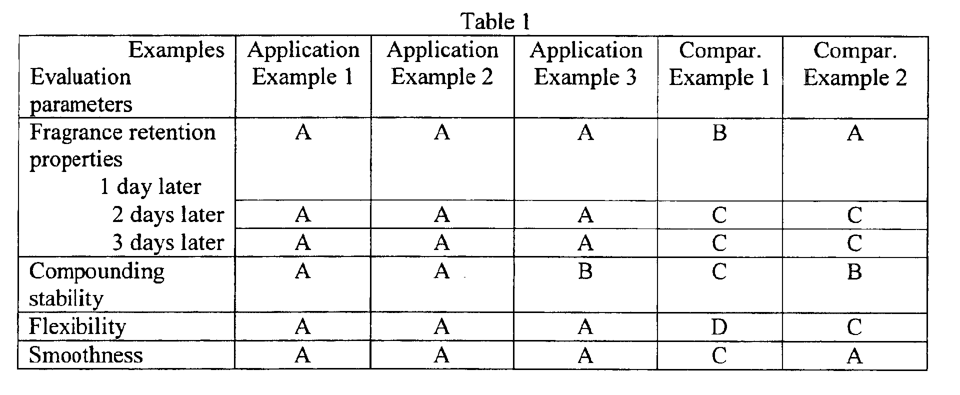

## Claims

1. An organopolysiloxane composition comprising (A) 5 to 99.9 wt% of a crosslinked product of a block copolymer consisting of an organopolysiloxane block and a polyoxyalkylene block bonded to silicon atoms and represented by the following general formula-R¹(OR²)ₘOR¹-, where R¹ and R² are independently selected alkylene groups and *m* is a positive integer and (B) 95 to 0.1 wt% of a fragrance material.

2. The organopolysiloxane composition according to claim 1, wherein at room temperature the crosslinked product of the block copolymer is in a liquid or a gel-like state.

3. The organopolysiloxane composition according to claim 1 or 2, wherein the crosslinked product is obtained by a hydrosilation reaction comprising
(a) an organopolysiloxane having at least two silicon-bonded hydrogen atoms in one molecule and
(b) a polyoxyalkylene of general formula R⁴(OR²)ₘOR⁴, where R² and *m* are as defined in claim 1 and each R⁴ is an independently selected alkenyl group.

4. The organopolysiloxane composition according to claim 1 or 2, wherein the crosslinked product is obtained by a hydrosilation reaction comprising
(a) an organopolysiloxane having at least two silicon-bonded hydrogen atoms in one molecule and
(b) a polyoxyalkylene of general formula R⁴(OR²)ₘOR⁴, where R² and *m* are as defined in claim 1 and each R⁴ is an independently selected alkenyl group, and
(c) an organopolysiloxane having at least two silicon-bonded alkenyl groups in one molecule.

5. The organopolysiloxane composition according to claim 1 or 2, wherein the crosslinked product comprises
(a) 1 part by weight organopolysiloxane having at least two silicon-bonded hydrogen atoms in one molecule,
(b) 0.01 to 100 parts by weight polyoxyalkylene of general formula R⁴(OR²)ₘOR⁴, where R² and *m* are as defined in claim 1 and each R⁴ is an independently selected alkenyl group,
(c) 0 to 100 parts by weight organopolysiloxane having at least two silicon-bonded alkenyl groups in one molecule, and
(d) a catalytic amount of a hydrosilation reaction catalyst.

6. The organopolysiloxane composition according to claim 5, wherein component (b) is a polyoxyalkylene described by general formula R⁴(OC₂H₄)ₚ(OC₃H₆)_{q}OR⁴, where R⁴ is the same as above, *p* is 0 or a positive integer and *q* is a positive integer, with the proviso that *p* is less than *q*.

7. The organpolysiloxane composition according to any of claims 1 to 6, which is emulsified in water.

8. A method for preparing an organopolysiloxane composition comprising crosslinking an emulsion comprising
(A) 5 to 99.9 wt% of a crosslinkable organopolysiloxane composition comprising
(a) 1 part by weight organopolysiloxane having at least two silicon-bonded hydrogen atoms in one molecule,
(b) 0.01 to 100 parts by weight polyoxyalkylene of general formula R⁴(OR²)ₘOR⁴, where R² is an alkylene group, each R⁴ is an independently selected alkenyl group, and m is a positive integer,
(c) 0 to 100 parts by weight organopolysiloxane having at least two silicon-bonded alkenyl groups in one molecule, and
(d) a catalytic amount of a hydrosilation reaction catalyst,
(B) 95 to 0.1 wt% of a fragrance material, and
(C) water.

9. The method for preparing an organopolysiloxane composition according to claim 8, wherein components (a), (b) and (B) are emulsified in water and then component (d) is added to effect crosslinking.

10. The method for preparing an organopolysiloxane composition according to claim 8, wherein components (a), (b), (c) and (B) are emulsified in water and then component (d) is added to effect crosslinking.

11. A method for preparing an organopolysiloxane composition comprising crosslinking an emulsion comprising
(A) 5 to 99.9 wt% of a crosslinkable organopolysiloxane composition comprising
(a) 1 part by weight organopolysiloxane having at least two silicon-bonded hydrogen atoms in one molecule,
(b) 0.01 to 100 parts by weight polyoxyalkylene of general formula R⁴(OR²)ₘOR⁴, where R² is an alkylene group, each R⁴ is an independently selected alkenyl group, and m is a positive integer,
(c) 0 to 100 parts organopolysiloxane having at least two silicon-bonded alkenyl groups in one molecule, and
(d) a catalytic amount of a hydrosilation reaction catalyst, and
(B) water; and adding thereto
(C) 95 to 0.1 wt% of a fragrance material.
